(19) Europäisches Patentamt
European Patent Office
Office européen des brevets

(11) Veröffentlichungsnummer: **0 332 094**
**A1**

# (12) EUROPÄISCHE PATENTANMELDUNG

(21) Anmeldenummer: **89103832.5**

(22) Anmeldetag: **04.03.89**

(51) Int. Cl.[4]: **A61K 9/22**

(30) Priorität: **08.03.88 DE 3807523**

(43) Veröffentlichungstag der Anmeldung:
**13.09.89 Patentblatt 89/37**

(84) Benannte Vertragsstaaten:
**AT BE CH DE ES FR GB GR IT LI LU NL SE**

(71) Anmelder: **BOEHRINGER INGELHEIM VETMEDICA GMBH**

**D-6507 Ingelheim/Rhein(DE)**

(72) Erfinder: **Kern, Otto, Dr.**
**Rinderbachstrasse 33**
**D-6507 Ingelheim am Rhein(DE)**
Erfinder: **Entenmann, Günther, Dr.**
**Schützenpfad 16**
**D-6507 Ingelheim am Rhein(DE)**
Erfinder: **Stricker, Herbert, Prof. Dr.**
**Richard-Lenel-Weg 13**
**D-6903 Neckargemünd(DE)**

## (54) Oral verabreichbares System zur kontrollierten Freigabe von Wirkstoffen.

(57) Die Erfindung betrifft ein oralverabreichbares Wirkstoffabgabesystem in Form eines "Bolus", daß sich nach einer vorherbestimmbaren Zeitspanne in kleine Bestandteile auflöst.

**EP 0 332 094 A1**

## Oral verabreichbares System zur kontrollierten Freigabe von Wirkstoffen

Die Erfindung betrifft ein oral verabreichbares System zur kontrollierten Freigabe von Wirkstoffen.

In der Tierhaltung müssen Tieren zu bestimmten Zwecken gewisse Stoffe über einen längeren Zeitraum zugeführt werden, so daß eine jedesmalige (z.B. tägliche) Gabe aus Gründen des Praktikabilität ausscheidet. Wegen der Art der Fütterung bzw. des Futters (z.B. Weidetiere) ist auch eine kontinuierliche Verabreichung durch Untermischung (z.B. unter ein Mehlfutter) oft nicht möglich. Letztgenannte Art der Verabreichung ist außerdem mit Ungenauigkeiten in der Dosierung behaftet, besonders bei Behandlung ganzer Tiergruppen und in ausgesprochen starkem Maße dann, wenn durch Beeinträchtigung des Appetits nur geringe und wechselnde Futtermengen aufgenommen werden. Beispiele für über einen längeren Zeitraum in therapeutischer oder prophylaktischer Absicht zu verabreichende Stoffe sind z.B. Antiparasitika, Antiinfektiva, stoffwechselregulierende oder die Gewichtszunahmen fördernde Stoffe. Zur kontrollierten Zufuhr solcher Stoffe über eine längere Zeit wurden verschiedene Systeme entwickelt, welche die Aufenthaltszeit eines Wirkstoffträgers im einhöhligen Magen oder in einem Teil des mehrhöhligen Magens von Wiederkäuern verlängern. Eine weitere Möglichkeit zur Verlängerung der Aufenthaltszeit eines Wirkstoffsystems in einem der Wiederkäuervormägen (Haube, Pansen) besteht in der Ausbildung des Systems als metallhaltiger Bolus. Ein solcher aus einem Kern und einer Hülle bestehender Bolus ist z.B. in der europäischen Patentanmeldung Nr. 0164241 beschrieben. Der Kern besteht aus einem bioabbaubaren Polymer und einem oder mehreren Wirkstoffen. Unter Abbau der Polymermatrix wird der Wirkstoff kontinuierlich freigesetzt. Die Hülle besteht aus einer mit Öffnungen versehenen Metallhülse.

Das Metall verhindert durch seine hohe Dichte den Weitertransport des Wirkstoffsystems in Richtung Darm ebenso wie den Rücktransport in das Maul beim Wiederkäuen, die Öffnungen gestatten den Austritt der aus dem Kern freigesetzten Wirkstoffe in die Magenflüssigkeit. Zahlreiche verschiedene Ausführungsformen der Kombination Metall / Wirkstoffträger sind bekannt. Mit diesen Systemen läßt sich im Pansen von Rindern über eine längere und durch besondere Ausführungsformen steuerbare Zeit eine konstant bleibende Wirkstoffmenge freisetzen. Nachteilig an allen diesen Systemen ist der Verbleib der Metallteile im Magen der Tiere. Bei Mehrfachanwendungen solcher Systeme kommt es zur Ansammlung erheblicher Metallmengen. Zudem wird Weidetieren zum Schutz vor unabsichtlich aufgenommenen spitzen Eisenteilen schon von vornherein ein Permanentmagnet in den Netzmagen (Haube) eingebracht. Bestehen nun die oben beschriebenen Wirkstoffsysteme aus magnetisierbarem Metall, werden diese Permanentmagnete durch die Wirkstoffsysteme blockiert und können ihre Schutzfunktion nicht mehr erfüllen. Werden die Metallteile der Wirkstoffsysteme aus nicht magnetisierbaren Legierungen hergestellt, so besteht die Gefahr der Freisetzung toxischer Metallionen (z.B. Blei) oder die Systeme werden durch die Verwendung korrosionsfester eisenfreier Legierungen sehr teuer.

Es ist die Aufgabe der vorliegenden Erfindung, ein Wirkstoffabgabesystem zur Verfügung zu stellen, welches

- im Magen von Wiederkäuern festgehalten wird,
- seine Wirkstoffe in vorgegebener Zeit kontrolliert abgibt,
- nach Abgabe der Wirkstoffe nicht im Magen verbleibt und
- nicht toxisch ist.

Diese Aufgabe wird erfindungsgemäß durch das nachfolgend beschriebene Wirkstoffabgabesystem (Bolus) gelöst. Das erfindungsgemäße Wirkstoffabgabesystem besteht aus Teilchen hoher Dichte, die zur Beschwerung dienen und durch einen in vorher bestimmbarer Zeit zerfallenden physiologisch unbedenklichen Hilfsstoff zusammengehalten werden und einen wirkstoffhaltigen Teil (Wirkstoff-Depot) und gegebenenfalls einem oder mehreren Hilfsteilen.

Das Teil mit hoher Dichte (im weiteren Text auch dichtegebendes Teil genannt) bewirkt, daß das erfindungsgemäße Wirkstoffabgabesystem für eine vorherbestimmbare Zeit im Magen des Wiederkäuers verbleibt.

Die Teilchen, bevorzugt pulverförmige Teilchen, bestehen aus Stoffen hoher Dichte und werden im weiteren Text auch Beschwerungsmittel genannt. Diese Stoffe sind bezüglich ihrer spezifischen Dichte, ihrer Zusammensetzung und ihrer Menge so auszuwählen, daß das Wirkstoffabgabesystem trotz der natürlichen Regurgitation im Magen verbleibt. In Abhängigkeit von der Größe des Wirkstoffabgabesystems und seiner Oberfläche kann die spezifische Dichte beliebige Werte über 1 g $cm^{-3}$ annehmen, bevorzugt wird jedoch ein Wert größer als 2,3 g $cm^{-3}$ . Da die Dichte des Hilfsstoffes üblicherweise zwischen 0,9 $g.cm^{-3}$ und 1,3 $g.cm^{-3}$ liegt, ist die Dichte des auszuwählenden Stoffes je nach Zusammensetzung des dichtegebenden Teiles im allgemeinen größer als 3,0 $g.cm^{-3}$, vorzugsweise größer als 4,0 $g.cm^{-3}$.

Nach oben stellt die Dichte kein einschränkendes Kriterium dar.

Beispielhaft, ohne die Erfindung jedoch einzuschränken werden Beschwerungsmittel aufgeführt, welche eine zur Herstellung eines dichtegebenden Teils ausreichend hohe Dichte aufweisen:

| | |
|---|---|
| Silber | 10,5 $g.cm^{-3}$ |
| Kupfer | 8,9 $g.cm^{-3}$ |
| Eisen | 7,8 $g.cm^{-3}$ |
| Nickel | 8,9 $g.cm^{-3}$ |
| Blei | 11,3 $g.cm^{-3}$ |
| Antimon | 6,7 $g.cm^{-3}$ |
| Zinn | 7,3 $g.cm^{-3}$ |
| Zink | 7,1 $g.cm^{-3}$ |
| Hydroxylapatit | 3,1-3,3 $g.cm^{-3}$ |
| Bariumsulfat | 4,5 $g.cm^{-3}$ |
| Eisenoxide | 5,2-5,7 $g.cm^{-3}$ |
| Bariumtitanat | 6,1 $g.cm^{-3}$ |
| Aluminiumoxid | 4,0 $g.cm^{-3}$ |
| Zinnoxid | 7,0 $g.cm^{-3}$ |
| Titandioxid (Rutil) | 4,2 $g.cm^{-3}$ |
| Mineralien wie z.B. | |
| Scheelit (Calciumwolframat) | 6,1 $g.cm^{-3}$ |
| Ferberit (Eisenwolframat) | 7,2 $g.cm^{-3}$ |
| Fayalit (Eisensilicat) | 4,4 $g.cm^{-3}$ |
| Hercynit (Eisenaluminiumoxid) | 4,3 $g.cm^{-3}$ |
| Powellit (Calciummolybdat) | 4,3 $g.cm^{-3}$ |
| Cacliumphosphate | |

Bevorzugt eingesetzt werden nicht toxische Stoffe, wie z.B. Bariumsulfat oder Eisenoxide.

Die Dichteangaben sind als Näherungswerte zu verstehen. Insbesondere bei mineralischen Stoffen können die Werte, z.B. durch den Gehalt an weiteren Stoffen, nach oben oder unten abweichen. Diese Stoffliste ist nicht einschränkend. Zahlreiche andere Stoffe aus den Gruppen der Metalle, Metalloxide, Salze und Mineralien weisen eine ausreichend hohe Dichte auf und sind zur erfindungsgemäßen Verwendung geeignet, soweit nicht toxikologische Erwägungen entgegenstehen.

In einer Ausführungsform sind die pulver- bzw. granulatförmigen Metalle bzw. Metallverbindungen, die als Beschwerungsmittel eingesetzt werden, mit einem nicht abbaubaren Polymer überzogen. Ein Vorteil dieser Ausführungsform ist es, daß auch Verbindungen als Beschwerungsmittel eingesetzt werden können, die bezüglich ihrer physiologischen Wirkungen nicht ganz unproblematisch sind, da die überzogenen Teilchen nicht mehr in direkten Kontakt mit den Verdauungssäften kommen. Geeignete Polymere sind hierzu bekannt, wie beispielsweise Polyethylen u.a. Geeignete Überzüge können beispielsweise durch Sprühen einer entsprechenden Lösung des Polymeren hergestellt werden.

Die Teilchengröße der Stoffe, die zur Beschwerung eingesetzt werden können, ist nicht kritisch, sollte aber so gewählt werden, daß die Teilchen nach dem Abbau des verbindenden Hilfsstoffes weiterbefördert werden können. Dies ist unter anderem von der spezifischen Dichte der Teilchen abhängig. Je höher die spezifische Dichte ist, desto kleiner sollten die Teilchen sein.

Während bei manchen Stoffen erheblich größere Teilchen noch akzeptabel sind, werden bei Stoffen hoher spezifischer Dichte Teilchengrößen kleiner als 1 mm, eventuell auch kleiner als 0.1 mm bevorzugt. Die Form und die Oberflächenbeschaffenheit der Teilchen ist nicht kritisch. Die Teilchen können kugelförmig oder stäbchenförmig sein oder eine beliebige andere Form aufweisen. Die Oberfläche kann glatt, rauh oder porös sein. In bestimmten Fällen ist es zweckmäßig, die Haftung zwischen den Teilchen hoher Dichte und dem sie verbindenden Hilfsstoff durch eine besondere Form (z.B. Sternform) oder eine besondere Oberflächenstruktur (z.B. Porenstruktur) zu verbessern.

Der Hilfsstoff der den Zusammenhalt der Teilchen hoher Dichte bewirkt, besteht aus einem Stoff, welcher nach einer bestimmten Zeit durch den Einfluß des Magensafts abgebaut wird und durch diesen Abbau den Zerfall des dichtegebenden Teils herbeiführt. Geeignet hierzu sind alle Stoffe, die nach einer bestimmten Zeit durch den Magensaft aufgelöst werden. Bevorzugt sind hierbei biologisch abbaubare Polymere. Solche Polymere sind in größerer Zahl bekannt, z.B. Polycyanacrylate, Polymethacrylate, Polyacrylate, Polyorthoester, Polyglycole, Polyphosphazene, modifizierte Stärke und Cellulose sowie natürliche Polymere wie Eiweiße (Kollagan, Gelatine), Stärke, Cellulose usw.. Geeignete Polyester bzw. Polylactone sind beispielsweise Polyglycolid, Polylactide, Polyhydroxybuttersäure, Polycaprolacton, Polydioxanon, Polytrimethylencarbonat, Polymere aus verschieden substituierten Glycoliden wie Diethylglycolid, Dipropylglycolid, Methylethylglycolid usw. sowie statistische Copolymere und Blockcopolymere aus den Monomeren Glycolid, Lactid, Dioxanon, Trimethylencarbonat, Caprolacton und anderen Monomeren.

Bevorzugt werden Polymere deren Abbau möglichst wenig durch Enzyme oder durch eine Veränderung des pH-Wertes beeinflußt wird. Bevorzugt werden außerdem Polymere, deren Abbau durch Wahl bestimmter Molekulargewichte und/oder Comonomerzusammensetzungen beeinflußt werden und deren Abbauprodukte nicht toxisch sind. Dies sind im besonderen Polymere und Copolymere des L-Lactids, D-Lactids, D,L-Lactids, meso-Lactids, Glycolids, Dioxanons und Trimethylencarbonats gemäß folgender Struktur

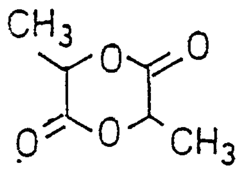

L-Lactid

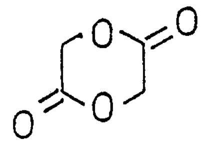

Glycolid

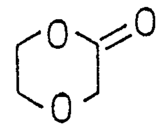

Dioxanon

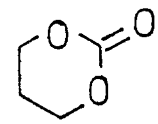

Trimethylen-carbonat

D-Lactid
D,L-Lactid
meso.Lactid

Besonders bevorzugt werden

Poly (L-lactid), Poly(D,L-lactid) oder Polyglycolid mit Molekulargewichten (bestimmt durch Endgruppentitration) kleiner 10000 für Zerfallszeiten des dichtegebenden Teils innerhalb weniger Wochen;

Poly (L-lactid), Poly (D,L-lactid) oder Polyglycolid mit inhärenten Viskositäten größer als 0,1 dl/g (Chloroform 25° C), vorzugsweise größer als 0,3 dl/g (Chloroform 25° C) für Zerfallszeiten innerhalb einiger Monate.

Die Polymere können in reiner Form, als Mischung untereinander oder als Mischung mit anderen abbaubaren oder nicht abbaubaren Polymeren eingesetzt werden.

Die Herstellung, Reinigung und analytische Charakterisierung der erfindungsgemäß bevorzugten Polymere ist in zahlreichen Patenten und Publikationen beschrieben. Beispielhaft seien genannt, DE-OS 2700729, US-PS 3912692 oder DE-OS 2850824.

Die Herstellung des dichtegebenden Teils erfolgt in an sich bekannter Weise durch Heißverpressen, Extrusion oder Spritzgußverarbeitung von Gemischen aus dem Beschwerungsmittel und einem polymeren Hilfsstoff. In einer anderen Ausführungsform wird das Beschwerungsmittel in einen Hohlkörper eingefüllt, welcher ganz oder teilweise aus einem abbaubaren Kunststoff besteht. Der Anteil an Beschwerungsmittel ist so zu wählen, daß die Dichte des gesamten Wirkstoffabgabesystems bevorzugt größer als 2,3 $g.cm^{-3}$ ist. Bei den meisten Systemen wird dies erreicht, wenn die Dichte des dichtegebenden Teils größer als 3 $g.cm^{-3}$ beträgt. Ein Formkörper mit dieser Dichte kann z.B. durch Heißverpressen einer Mischung von 1 Gewichtsteil Poly(D,L-lactid) (Dichte 1,2 $g.cm^{-3}$) und 4,5 Gewichtsteilen Bariumsulfat (Dichte 4,5 $g.cm^{-3}$) erhalten werden. Der Formkörper kann eine beliebige Gestalt (Kugel, Zylinder, Scheibe usw.) haben, soweit er das Abschlucken durch das Tier nicht unmöglich macht und durch eine entsprechende Vorrichtung dem Tier eingegeben werden kann. Vorrichtungen zur Verabreichung eines solchen Formkörpers gehören zum Stand der Technik und bedürfen keiner weiteren Ausführungen. Bevorzugt wird eine zylindrische Form mit abgerundeten Enden (Bolus) gemäß Fig. 1 bis 12. Die Größe eines solchen Bolus ist in weiten Grenzen variierbar, wobei eine obere Grenze durch das Applikationsverfahren (durch den Schlund) und eine untere Grenze dadurch gegeben ist, daß der Bolus als ganzes Teil nicht ausgeschieden werden soll. Boli haben im allgemeinen eine Länge bis ca. 10 bis 12 cm und einen Durchmesser bis ca. 3 bis 4 cm.

Der wirkstoffabgebende Teil (Wirkstoff-Depot) kann dabei in Höhlungen des Wirkstoffabgabesystems gemäß Fig. 1 oder Fig. 2, in Form von Ringen gemäß Fig. 3 oder als umgewickelte Folie gemäß Fig. 4 mit dem Bolus verbunden werden. Bei Ausführungsformen gemäß Fig. 2 und Fig. 3 kann die Wirkstoffdosis in technologisch vorteilhafter Weise durch Variation der Anzahl wirkstoffabgebender Teil bei einheitlicher Konstruktion des dichtegebenden Teils (vgl. Fig. 3B) beeinflußt werden. In einer weiteren Ausführungsform besteht der dichtegebende Teil aus einem Behälter aus einem abbaubarem Polymer, welcher mit Teilchen

hoher Dichte gefüllt ist. Der Behälter kann als "Flasche/Deckel-Anordnung" gemäß Fig. 5, als offene "Flasche" gemäß Fig. 6 oder als doppelhälftige Kapsel gemäß Fig. 7 ausgeführt sein. Die Anbringung des wirkstoffabgebenden Teils ergibt sich aus Fig. 5-7.

In einer bevorzugten Ausführungsform besteht das erfindungsgemäße Wirkstoffabgabesystem aus einem verschließbaren Hohlkörper, der gleichzeitig die Funktion des dichtegebenden Teils übernimmt und einem dazugehörigen separaten Wirkstoff-Depot. Der Hohlkörper weist genügend Öffnungen auf, damit der Magensaft ungehindert an das eingeschlossene - eventuell mehrteilige - Wirkstoff-Depot vordringen kann, um den Wirkstoff aus dem Depot herauszulösen. Das Wirkstoff-Depot ist so ausgebildet, daß es durch eine verschließbare Öffnung des Hohlkörpers hindurch in diesen eingeführt werden kann und dort so lange verbleibt, bis es nach der vorher bestimmten Zeit abgebaut worden oder der Hohlkörper zerfallen ist. Diese Ausführungsform bietet den Vorteil, daß nur wenige verschieden große Boli notwendig sind (ca. 2 -3 verschiedene Größen), so daß jederzeit je nach Notwendigkeit die geeignete Therapie bezüglich des Krankheitsbildes und der benötigten Arzneimittelmenge in Abhängigkeit der Tiergröße vom Tierarzt zusammengestellt werden kann. Die Variationsbreite des Wirkstoffabgabesystems ergibt sich dadurch, daß eine Vielzahl von verschiedenen Wirkstoff-Depots (bezüglich Arzneimittel und Menge) zur Verfügung gestellt werden, die dann gegebenenfalls in einem Hohlkörper kombiniert werden können.

Einzelne Ausführungsformen dieser bevorzugten Ausführungsform sind nachfolgend beschrieben, ohne jedoch die Vielfältigkeit der Gestaltungsmöglichkeit des erfindungsgemäßen Systems zu beschränken. Einzelne Beispiele beschreiben die Abbildungen der Fig. 8 bis Fig. 12. Hierbei besteht das System aus einem mit Schlitzen oder Löchern versehenem zylinderförmigen Hohlköper aus abbaubarem Polymer, der mindestens ein abgerundetes Ende aufweist (s. Fig. 8a, 8b) und ein Gemisch aus einem abbaubaren Polymer und Teilchen (Beschwerungsmittel) hoher Dichte enthält (s. Fig. 9) oder ganz aus einem ein Beschwerungsmittel enthaltenen abbaubaren Polymer besteht (s. Fig. 10). Das offene Ende des Zylinders ist mit einem gegebenenfalls gleichfalls gefensterten oder durchlöcherten Verschluß verschließbar, der aus einem der beiden zuvor genannten Materialien besteht (s.Fig. 9 und 10).

Der Hohlraum des Bolus dient zur Aufnahme des Wirkstoffdepots, das in den verschiedensten Formen (z.B. glatte, gerollte oder gefaltete Filme, Plättchen, Stäbchen, Kugeln, Tabletten, Schläuche, Ringe usw. mit gleichartigem oder geschichtetem Aufbau) ausgebildet sein kann (s. Fig. 9 und 10). Durch Größe und/oder Anzahl dieser Körper läßt sich, entsprechend dem unterschiedlichen Körpergewicht der zu behandelnden Tiere die Dosierung des Wirkstoffs varrieren.

Bei einer weiteren Variante wird ein mit Öffnungen versehener halb kapselförmiger oder zylindrischer erfindungsgemäßer Hohlkörper ein- oder beidseitig mit einem Verschluß aus einer Mischung aus abbaubarem Polymer und Teilchen hoher Dichte verschlossen (s. Fig. 11 und 12), dessen Innenraum, wie oben beschrieben, die wirkstofftragenden Körper aufnimmt.

In einer weiteren Ausführungsform wird ein Kern aus dem dichtegebenden Teil gebildet, z.B. in Form einer Kugel, der dann von dem Wirkstoff-Depot umhüllt wird. Bei einer weiteren Ausführungsform besteht das Wirkstoffabgabesystem aus einem homogenen Polymerisat oder Copolymerisat, z.B. in Form einer massiven Kugel oder eines massiven Zylinders, in das sowohl das Beschwerungsmittel wie auch der Wirkstoff eingebettet sind.

Die wirkstoffabgebenden Teile (Wirkstoff-Depot) des Systems sollen einen oder meherere Wirkstoffe über einen längeren Zeitraum kontrolliert abgeben. Dabei ist es von Vorteil, wenn die Wirkstoff-Freigabe variabel ist, damit die Freisetzungsrate sowohl dem Wirkstoff als auch der Therapie angepaßt werden kann. Je nach Behandlungsziel und -dauer kann nämlich nicht nur eine gleichbleibende, sondern teilweise auch eine abnehmende oder sogar zunehmende Wirkstoffabgabe wünschenswert oder erfoderlich sein.

Ein Bestandteil dieser Erfindung ist es weiterhin, ein bei Wiederkäuern oral einsetzbares Wirkstoff-Depot mit kontrollierter verzögerter Wirkstofffreigabe zur Verfügung zu stellen, das sich nach Erschöpfen des Wirkstoffdepots zu Bestandteilen auflöst, welche auf natürlichem Weg ausgeschieden werden. Obwohl jedes Wirkstoff-Depot, das den Wirkstoff über einen längeren Zeitraum in einer kontrollierte Freigaberate abgibt und sich anschließend - nach erfolgter Wirkstoffabgabe - in kleine Bestandteile auflöst geeignet ist, ist ein Wirkstoff-Depot bevorzugt, bei dem das System aus einem oder mehreren Wirkstoffen und einem oder mehreren Polymeren besteht, welche unter dem Einfluß des Magensafts ebenso abbaubar sind wie die dichtegebenden Teile. Für die Auswahl des Polymers (im weiteren Text auch Trägermaterial genannt) bezüglich seiner Abbaubarkeit gelten daher die gleichen Kriterien wie bei den dichtegebenden Teilen des Systems. Da jedoch außer der Abbauzeit auch die Freigabecharakteristik von großer Bedeutung ist, kann es erforderlich sein, die Freigaberate und/oder die Abbaugeschwindigkeit durch Zusätze zu beeinflussen. So ist z.B. bei der Therapie akuter Erkrankungen eine Wirkstofffreigabe innerhalb von 1 bis 2 Wochen; zur Prophylaxe gegen Wurmbefall aber eine Wirkstofffreigabe über mehrere Wochen erforderlich. Wirkstoffabgabesysteme mit wachstumsfördernden Stoffen sollen ihre Funktionsfähigkeit sogar über Monate behalten.

Dies wird durch die Verwendung eines Polymers oder Copolymers aus L-Lactid, D-Lactid, D,L-Lactid, meso-Lactid, Dioxanon, Glycolid, Caprolacton oder Trimethylencarbonat als Trägermaterial im wirkstoffabgebenden Teil des Systems gelöst.

Bevorzugt wird hierbei Poly(D,L-lactid), ggf. mit definierten Anteilen an Zusätzen .

Geeignete Zusätze sind pharmakologisch verträgliche Lösungsmittel bzw. Weichmacher, bevorzugt ein Essigsäureester, mit einem Anteil bis zu 10 % und/oder einem biologischen abbaubaren niedermolekularem Polymer, bevorzugt Polymilchsäure, mit einem Anteil bis zu 60 % und/oder suspendierten, wasserlöslichen Porenbildnern, wie z.B. Lactose, mit einem Anteil bis zu 50 %.

Poly-D,L-Lactide sind in einem weiten Molekulargewichtsbereich bekannt. Für das erfindungsgemäße wirkstoffabgebende Teil sind besonders mittelmolekulare Poly-D,L-Lactid-Typen geeignet, die eine inhärente Viskosität zwischen 0.3 und 4.5 (gemessen in Chloroform bei 25° C) aufweisen.

In einer bevorzugten Ausführungsform besteht das Trägermaterial des erfindungsgemäßen Systems aus Poly-D,L-Lactid.

In einer anderen Ausführungsform besteht das erfindungsgemäße Trägermaterial aus einem Copolymer aus D,L-Lactid und Glycolid, wobei jedoch der Anteil des Glycolids an dem Copolymerisat 50 Gew. % nicht überschreiten sollte.

Überraschenderweise wurde gefunden, daß die Abbaurate des wirkstoffabgebenden Teils durch einen definierten Gehalt an Essigsäureester oder eines anderen physiologisch unbedenklichen Lösungsmittels oder eines Weichmachers bzw. eines Lösungsmittelgemisches, welche auch nach längerer Lagerung quantitativ im Polymer verbleiben, gesteuert werden kann. Dies ist von entscheidender Bedeutung, da einerseits das wirkstoffabgebende Teil ausreichend rasch abgebaut werden soll, andererseits ein zu schneller Abbau des wirkstoffabgebenden Teils zu einer unkontrollierten Freisetzung des Wirkstoffes führen würde. Der Gehalt an Essigsäureester kann bis zu 10 % betragen, wobei ein steigender Anteil Essigsäureester den Abbau des Poly-D,L-Lactids beschleunigt.

Überraschenderweise wurde gefunden, daß der Zusatz von Essigsäureester zwar die Abbaurate des wirkstoffabgebenden Teils beeinflußt, aber auf die Wirkstofffreigabe keinen nennenswerten Einfluß hat.

Essigsäureester im Sinne der Erfindung sind die $C_1$ - $C_5$ Alkylester der Essigsäure, wie z.B. der Methyl-, Ethyl-, n-Propyl-, n-Butyl, iso-Butyl-, tert-Butyl-, n-Pentyl-, sec-Pentyl-, iso-Pentyl und der tert.-Pentylester. Besonders bevorzugt ist der Essigsäureethylester, auch als Essigester bezeichnet.

In einer weiteren Ausführungsform kann das erfindungsgemäße wirkstoffabgebende Teil auch niedermolekulare Polymere, wie z.B. Poly(L-milchsäure), Poly(D-milchsäure), Poly(D,L-milchsäure), Poly-(glycolsäure), Poly(L-milchsäure-co-glycolsäure), Poly(D-milchsäure-co-glycolsäure), Poly(D,L-milchsäure-co-glycolsäure) enthalten. Bevorzugt werden Poly(L-milchsäure) und Poly(D,L-milchsäure). Die Molekulargewichte (bestimmt durch Endgruppentitration) betragen 500 bis 5000, vorzugsweise 1500 bis 2500.

Diese Zusätze ermöglichen für sich allein oder in Kombination mit einem Essigsäureester ebenfalls eine Steuerung der Abbaurate des wirkstoffabgebenden Teils.

Eine Beeinflussung der Wirkstofffreigabe kann auf verschiedene Weise vorgenommen werden:

a) Zusatz eines Porenbildners, wie z.B. Lactose u.a..

b) Zustand des Wirkstoffs (gelöst, suspendiert, Teilchengröße).

c) Verarbeitungsform des Trägers (Monolyt, Polydispers. Schichtaufbau).

Neben Verbindungen, die die Abbaurate des Trägermaterials beeinflussen, enthält somit das erfindungsgemäße wirkstoffabgebenden Teils auch Stoffe in Form von Porenbildnern, die eine Steuerung der Wirkstofffreigabe ermöglichen. Erfindungsgemäß geeignete Porenbildner sind beispielsweise wasserlösliche pharmazeutisch akzeptable Monosaccharide und Disaccharide. Bevorzugt wird Lactose, geeignet sind jedoch auch Glucose, Fructose, Xylose, Galactose, Sucrose, Maltose, Saccharose und verwandte Verbindungen wie Mannit, Xylit, Sorbit. Andere geeignete Hilfsstoffe sind Salze wie Lactate, Glyconate oder Succinate des Natriums, Kaliums oder Magnesiums. Eine rasche Freisetzung der Wirksubstanz von Anfang an (nach erfolgter Verabreichung) wird dann erreicht, wenn die Lösungsgeschwindigkeit des Porenbildners sehr viel größer ist als die Freisetzungsgeschwindigkeit der Wirksubstanz. Dies ist beispielsweise bei guter Löslichkeit und kleiner Partikelgröße des Porenbildners, z.B. Lactose, der Fall.

Eine verspätet beschleunigte Freisetzung der Wirksubstanz wird dann erreicht, wenn die Löslichkeit des Porenbildners sehr viel kleiner ist als die der Wirksubstanz; so z.B. bei schlechter Wasserlöslichkeit des Porenbildners. Durch die verspätet beschleunigte Freisetzung der Wirksubstanz wird erreicht, daß der lineare Freigabeverlauf der Wirksubstanz auch bei längerer Applikation gewährleistet bleibt.

Unter Ausnutzung der verschiedenen Parameter können wirkstoffabgebende Teile hergestellt werden,

die sowohl eine individuell einstellbare Freigabe- wie auch Abbaurate aufweisen.

Entsprechend den verschiedenen Ausführungsformen des dichtegebenden Teils in Fig. 1 bis 7 werden für die wirkstoffabgebenden Teile folgende Ausführungsformen bevorzugt:

A) massive, poröse oder mit Kanälen versehene Stäbchen für Ausführungsformen gemäß Fig. 1, oder Fig. 8 bis 12

B) gerollte Stäbchen (Filmrollen) für Ausführungsformen gemäß Fig. 1, oder Fig. 8 bis 12

C) Ringe für Ausführungsformen gemäß Fig. 3 und 7A

D) Kugeln, Halbkugeln oder anders geformte Stopfen für Ausführungs formen gemäß Fig. 5 oder Fig. 6

E) Schläuche/Röhren für Ausführungsformen gemäß Fig. 4 oder Fig. 7B.

F) Massive, poröse oder mit Kanälen versehene Kugeln, Halbkugeln, Scheiben, Granulate oder anders geformte Teilchen für Ausführungsformen gemäß Fig. 8 bis Fig 12.

Massive Stäbchen des Typs A können mehrschichtig aufgebaut und bespielsweise nach folgendem Verfahren hergestellt werden.

In dem gelösten Polymer, z.B. mit Essigester als Lösungsmittel, wird der Wirkstoff suspendiert und die erfindungsgemäßen Zusätze beigemengt. Wenn es gewünscht wird, können dem gelösten Polymer neben dem Wirkstoff und den Zusätzen noch weitere pharmazeutisch Hilfsstoffe zugesetzt werden. Danach wird die Suspension auf einer Fläche ausgegossen und zu einem Film getrocknet. Hierbei werden die Trocknungsbedingungen so eingestellt, daß die gewünschte Restmenge an Lösungsmittel in dem Polymer verbleibt im allgemeinen zwischen 1 und 7 %. Die getrockneten Filme haben eine Schichtdicke zwischen 30 und 1000 $\mu$, bevorzugt ca. 100 $\mu$. Geräte und Verfahren zur Herstellung solcher Filme sind dem Fachmann bekannt und bedürfen keiner weiteren Ausführungen. Es ist selbstverständlich, daß der Trocknungsprozeß mit einer gewissen Sorgfalt (langsam, geringe Temperatur-, Vakuum-, und Feuchtigkeitsschwankungen) durchgeführt werden muß, damit die Filme plan bleiben.

Mehrschichtige Filme können durch ein erneutes Aufbringen von Polymerlösung (mit oder ohne Wirkstoff) erhalten werden.

Nachdem der Film getrocknet ist, wird er zu Stäbchen der gewünschten Länge geschnitten.

Stäbchen des Typs B bestehen aus einem oder mehreren aufgerollten ein- oder mehrschichtigen Polymerfilmen.

Das erfindungsgemäße wirkstoffabgebende Teil des Typs B wird ebenfalls aus wirkstoffhaltigen Filmen hergestellt, wobei jedoch die Dicke der Filme wesentlich geringer ist, im allgemeinen zwischen 30 und 500 $\mu m$, bevorzugt 70 bis 90 $\mu m$. Nach dem Trocknen werden die Filme geschnitten und zu Stäbchen gewünschten Durchmessers, bis ca. 3 mm, gerollt, die dann auf die gewünschte Länge geschnitten werden. Die Stäbchen können so aufgerollt werden, daß der Kern einen Hohlraum enthält. Bei dem Laminat des Typs B können auch mehrere Filme übereinandergegossen oder bevorzugt übereinander gegossen werden, die dann zu einem Stäbchen gerollt werden. Durch die Kombination mehrerer Filmschichten können in einfacher Weise Wirkstoffe kombiniert werden und Schichten unterschiedlicher Wirkstoffkonzentration hergestellt werden. Die einzelnen Schichten können verschiedene Freisetzungsgeschwindigkeiten aufweisen.

Neben einer alternierenden Schichtfolge ist es auch möglich, zuerst einen aufgerollten Kern zu bilden und anschließend außen weitere Filmschichten aufzubringen.

Durch die Verwendung von Filmschichten mit unterschiedlicher Freisetzungscharakteristik können auch verschiedene Wirkstoffe in zeitlich voher bestimmter Reihenfolge abgegeben werden. Es ist nicht unbedingt erforderlich, daß alle Filmschichten Wirkstoffe enthalten.

Bei der Herstellung des erfindungsgemäßen wirkstoffabgebenden Teils des Typs B sollten die Filme einen relativ hohen Gehalt an Restlösungsmittel aufweisen (ca. 10.%), wenn sie gerollt werden. Hierdurch wird vermieden, daß die Filme brüchig werden. Das fertig gerollte Stäbchen wird dann noch einmal einem Trocknungsprozeß unterworfen, um den gewünschten Gehalt an Restlösungsmittel einzustellen.

Ringe des Typs C können durch Ausstanzen aus ein- oder mehrschichtigen Filmen erhalten werden, wie sie bei der Herstellung von wirkstoffabgebenden Teilen des Typs A oder B anfallen. Sie können ebenso wie die wirkstoffabgebenden Teile des Typs A, D, E oder F vorteilhaft durch Extrusion von Granulaten aus Wirkstoff und Polymer oder Copolymer, gegebenenfalls mit Zusätzen, wie z. B. Polymilchsäure oder einem Porenbildner, wie z.B. Lactose hergestellt werden. Die Herstellung von Formkörpern des Typs A, C, D, E, F kann auch durch Verpressen einer Mischung aus Wirkstoff und Polymer oder Copolymer, gegebenenfalls mit Zusätzen hergestellt werden, wobei Druck, Temperatur und Presszeit so einzustellen sind, daß die gewünschte mechanische Festigkeit erreicht wird.Die bisherigen Befunde zeigen, daß die nach der "Lösungsmittelmethode" hergestellten wirkstoffabgebenden Teile ein anderes Abbauverhalten zeigen als

die Extrudate, d.h. die Extrudate werden bei gleicher Polymerzusammensetzung langsamer abgebaut (vgl. Abb. I). Der Unterschied beruht darauf, daß aufgrund der relativ hohen Temperaturen beim Extrusionsvorgang ein definierter, höherer Gehalt an Restlösungsmittel nicht eingestellt werden kann.

In einer anderen Ausführungsform ist das Wirkstoff-Depot aus kleinen separaten wirkstoffhaltigen Teilchen gebildet, die ebenfalls eine verzögerte Freisetzung des Wirkstoffs bewirken, jedoch nach der Wirkstofffreigabe nicht abgebaut werden. (Eine verzögerte Freisetzung kann beispielsweise durch entsprechende Überzüge oder Trägermaterialien aus emulsionspolymerisierten Polyacrylaten (Eudragit®) erzielt werden). Dies setzt voraus, daß die wirkstoffhaltigen Teilchen so klein sind, daß sie nach dem Abbau des Bolus problemlos ausgeschieden werden können. Bei dieser Ausführungsform ist der Bolus so auszugestalten, daß der Magensaft zwar an die Teilchen gelangen kann, die entsprechenden Öffnungen im Bolus jedoch so dimensioniert sind, daß die Teilchen so lange im Bolus verbleiben, bis er abgebaut ist.

Geeignete Wirkstoffe sind solche, die in dem Polymer in suspendierter Form vorliegen. Besonders geeignet sind z.B. die wasserlöslichen Salzformen von Basen, wie z.B. Hydrochloride oder Hydrobromide. Ganz besonders geeignet ist Clenbuterolhydrochlorid.

Weiterhin können im Anwendungsbereich der Veterinärmedizin die nachfolgend genannten Stoffgruppen und Verbindungen in den erfindungsgemäßen Freigabesystemen eingesetzt werden.

- Glucocorticoide zur Geburtsinduktion, z. B. Dexamethason, Betamethason, Flumethason, deren Ester.und Derivate,
- Gestagene zur Brunstsynchronisation, Brunst- und Läufigkeitsunterdrückung,
- $\beta_2$-Adrenergika zur Therapie und Prophylaxe von Respirationserkrankungen, zur Verhinderung von Abort und Geburt, zur Wachstumsförderung und Stoffwechselbeeinflussung, wie z. B. Clenbuterol, 4-(2-tert.-Butylamino-1-hydroxyethyl)-2-cyano-6-fluor-phenylcarbaminsäure-ethylester-hydroclorid, α-[[[3-(1-Benzimidazolyl)-1,1-dimethylpropyl]-amino]-methyl]-2,-fluor-4-hydroxy-benzyl alkohol-methansulfanat monohydrat, (Cimaterol), 1-(4-Amino-3-cyanophenyl)-2-iso-propylaminoethanol,
- ß-Blocker zur Reduzierung von Transport-Stress, $\alpha_2$-Adrenergika gegen enteritische Erkrankungen und zur Behandlung hypoglykämischer Zustände, sowie zur Sedation (z. B. Clonidin, 2-[2-Brom-6-fluorphenylimino]-imidazolidin,
- Benzodiazepine und Derivate, wie z.B. Brotizolam zur Sedation,
- Antiphlogistika zur antiinflammatorischen Therapie, z.B. Meloxicanm
- Endorphine zur Anregung der Pansenmotorik,
- Steroidhormone (natürliche und synthestische) zur Wachstumsförderung, z.B. Östradiol, Progesteron und deren Ester und synthetische Derivate wie z.B. Trenbolon,
- Antiparasitika zur Bekämpfung von Endo- und Entoparasiten, wie z.B. Levamisol, Avermectin, Benzimidazole, Pyrantel, Morantel, Febantel,
- Herz- und Kreislaufaktive Substanzen, z.B. Etilefrin oder Pimobendan.

Die Herstellung von erfindungsgemäßen Wirkstoff-Abgabe-Systemen aus dichtegebenden Teilen und wirkstoffabgebenden Teilen ergibt sich aus Fig. 1 bis Fig. 12:

Im Beispiel nach Fig. 1 und Fig. 2 werden Stäbchen nach Typ A oder Typ B in Bohrungen des dichtegebenden Teiles eingefügt.

In Beispielen nach Fig. 3A, 3B und 7A werden Ringe des Typs C über Formkörper mit entsprechenden Einkerbungen gezogen.

In Beispielen nach Fig. 4 und Fig. 7B wird das dichtegebende Teil in einen Schlauch des Typs E eingeführt.

Wirkstoffabgabesysteme gemäß Fig. 4 können auch durch Coextrusion hergestellt werden.

Bei der Herstellung von Wirkstoffabgabesystemen gemäß Fig. 5 - 7B werden zunächst durch Spritzguß die Behälter hergestellt, mit Teilchen hoher Dichte gefüllt und anschließend gemäß Fig. 5 oder 6 verschlossen.

Die erfindungsgemäßen Ausführungsformen gemäß den Figuren 8 bis 12 können nach bekannten Verfahren zur Herstellung solcher zylinderartigen Formen hergestellt werden. Nach dem die fertigen Formen mit dem Wirkstoff-Depot gefüllt sind, werden sie mit einem Deckel verschlossen.

Obwohl das erfindungsgemäße Wirkstoffabgabesystem bevorzugt in Widerkäuern eingesetzt werden kann, ist in besonderen Fällen auch ein Einsatz bei anderen Tierarten vorteilhaft.

Nachfolgend wird die Erfindung an Beispielen näher erläutert.

In den Beispielen werden die nachfolgenden Polymere eingesetzt

An Polymermaterialien finden Verwendung:

D,L-Polylaktid I [η] = 1,0 ($\frac{100\ ml}{g}$) = MG* = 123.000

D,L-Polylaktid II [η) = 2,2 " = MG = 300.000

D,L-Polylaktid III MG = 11.500

D,L-Polymilchsäure (MG = 2000)

η = Grenzviskosität (intrinsic viscosity)

* bestimmt durch Gasphasenchromatographie (Standard: Polystyrol)

Beispiel 1:

(Faktoren des Polymerabbaues: Verarbeitungsmethode, Taktizität, Molmasse)

25 g D,L-Polylaktid werden in 75 g Essigester gelöst und mit einem Rakel auf einem glatten Untergrund zu einem Film ausgezogen. Nach Trocknung über mind. 24 Stunden wird dies zwei bzw. dreimal wiederholt, bis ein Mehrschichtenfilm von 250 µ Dicke entstanden ist. Anschließend wird der Film zuerst bei 23° C, dann bei 40° C im Vakuum bis zu einem vorgegebenen Lösungsmittelrestgehalt getrocknet, in 3 x 2,5 cm Stücke geschnitten und zu Rollen (Länge 3 cm, θ 2,8 mm) geformt.

Mittels Lösungsmethode hergestellte wirkstoffabgebende Teile zeigen hinsichtlich Molmassenabnahme in einer Pufferlösung ein anderes Verhalten, als z.B. durch Extrusion gewonnene Teile, d.h., sie werden vorteilhafterweise rascher abgebaut (Abb. 1). Die Taktizität des Polymeren spielt bei der Abbaugeschwindigkeit eine größere Rolle als die molare Masse bzw. Grenzviskosität [η] (Abb. 2). Hingegen spielt der pH-Wert der Lösung keine rolle, vgl. z.B. Makino et al., J. Mircoencasul. 3, 203-212 (1986).

Eine signifikante Massenabnahme setzt nach ca. 70 Tagen ein, d.h. nachdem die Grenzviskosität auf einen Wert von [η] 0,3 (100 ml/g) abgesunken ist.

Beispiel 2:

(Faktoren des Polymerabbaues: Essigesterrestgehalt, Polymilchsäurezusatz)

Mehrschichtenfilmrollen werden, wie in Beispiel 1 beschrieben, hergestellt, wobei im Falle der Charge I 50 % des D,L-Polylaktids durch D,L-Polymilchsäure (Molmasse 2000) ersetzt werden.

Abb. 3 zeigt, daß die Molmassenabnahme in wäßrigem Medium durch 4 bzw. 7 % Essigesterrestgehalt beschleunigt wird, nicht jedoch durch 1 %. Sehr ausgeprägt in dieser Beziehung wirkt der 50 %ige Zusatz von D,L-Polymilchsäure.

Die Massenabnahme verhält sich zur Molmassenabnahme wie in Beispiel 1 beschrieben (Abb. 4).

Beispiel 3:

(Faktor der Substanzfreigabe: Laktosezusatz)

8,8 g D,L-Polylaktid II ([η] = 2,2 (100 mlg/g)) werden in 45 g Essigester gelöst und 2,7 g Clenbuterol. HCl (20 µ = x = 53 µm) suspendiert, und ein Dreischichtenfilm entsprechend Beispiel 1 hergestellt. Im Falle der Charge L wird in der Polymerlösung für die mittlere Schicht zusätzlich 25 Gew.% Laktose (1 - 5 µm) suspendiert.

Abb. 5 zeigt, daß sich durch den Laktosezusatz die Clenbuterolfreigabe in wäßrigem Medium beschleunigen und damit steuern läßt.

Beispiel 4:

(Faktor der Substanzfreigabe: Polymilchsäurezusatz)

Die Dreischichtenfilmrolle L aus Beispiel 4 wird mit einer analog hergestellten Zubereitung verglichen, bei der 25 % des D,L-Polylaktids II durch D,L-Polymilchsäure (Molasse 2000) ersetzt ist.

Während die Clenbuterolfreigabe in wäßrigem Medium durch den Polymilchsäurezusatz stark beschleunigt wird, blieb der Essigesterrestgehalt im Bereich von 1 - 4 % ohne Effekt auf das Freigabeverhalten.

Polymilchsäure kann demnach ebenso wie Laktose als ein die Freigabe steuernder Zusatz eingesetzt werden (Abb.6).

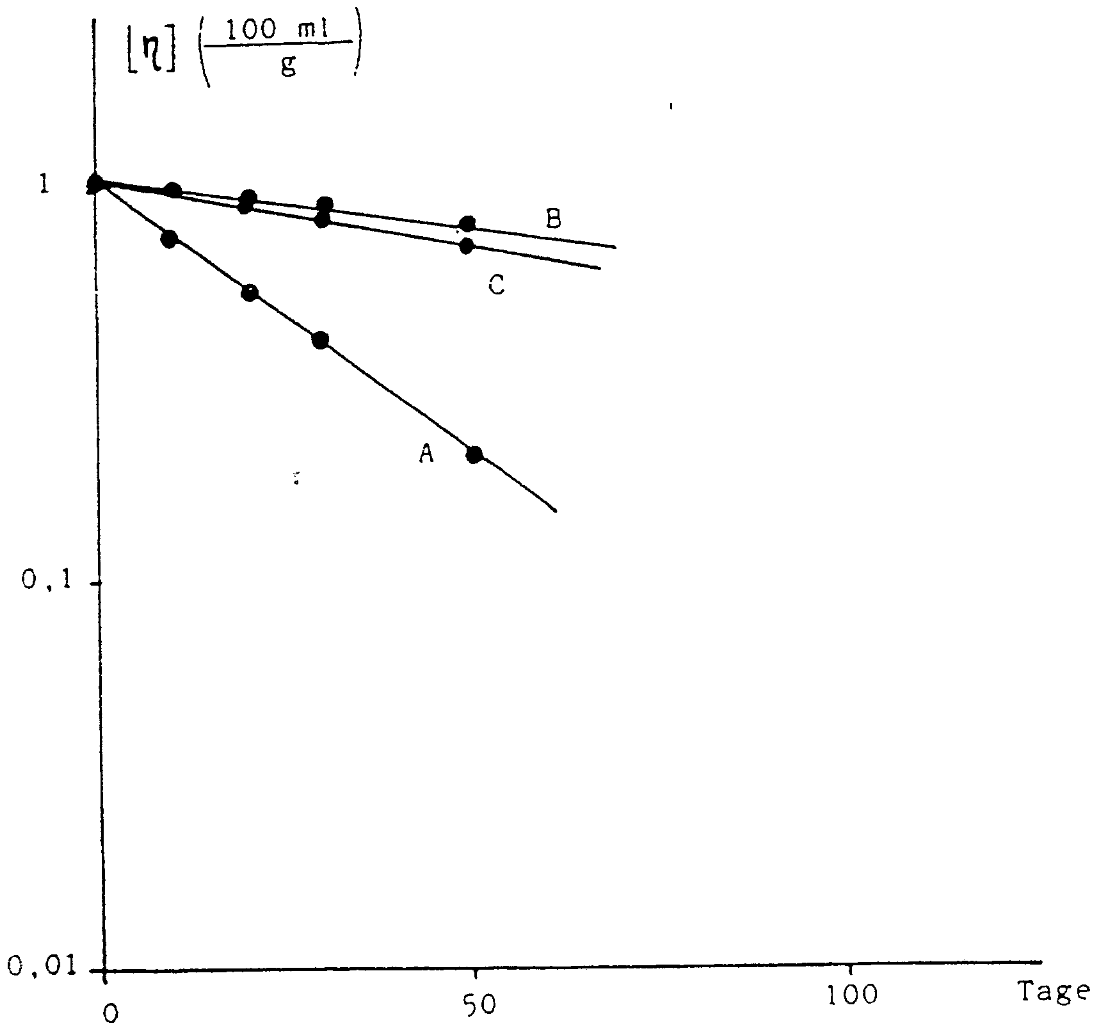


Abb. 1: Molmassenabnahme von Polylaktid-Formkörpern

[η] = Grenzviskositat
Versuchsbedingungen: isoton. Phosphatpuffer PH 7,4; 37° C
A: Gerolltes Stäbchen aus D,L-Polylaktid I (Lösungsmethode
B: Extrudatzylinder aus D,L-Polylaktid I
C: DL-Polylaktid I-Pulver

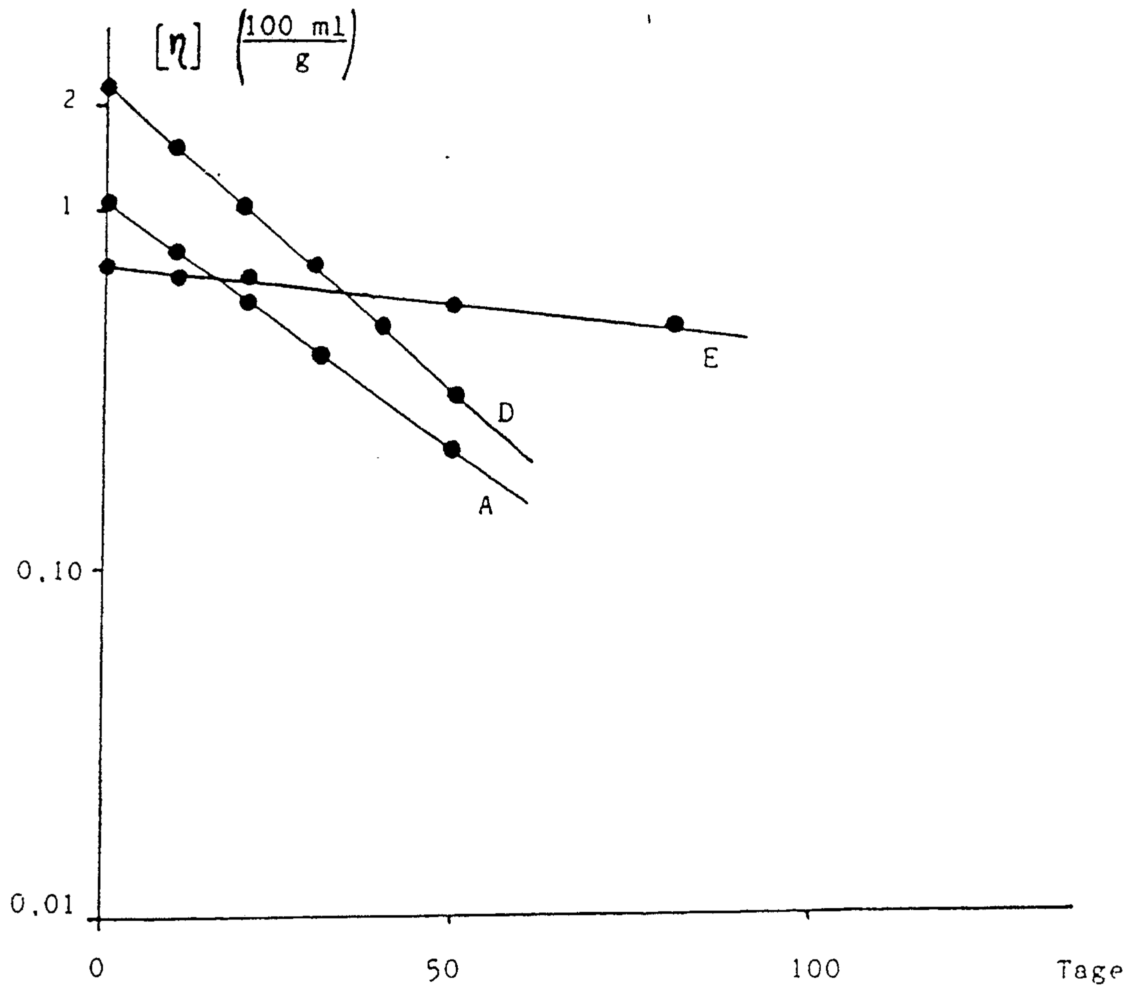


Abb. 2 Molmassenabnahme von Polylaktid-Formkörpern

Versuchsbedingungen: isotonischer Phosphatpuffer pH 7,4; 37° C
Zubereitung: Gerollte Stäbchen aus Polylaktid (Lösungsmethode)
A: D,L-Polylaktid I; 7 % Essigester, Tg = 26° C
D: D,L-Polylaktid II; 7 % Essigester T = 30° C
E: L-Polylaktid; 7 % Essigester, Fp = 172° C
(Vergleichsbeispiel)

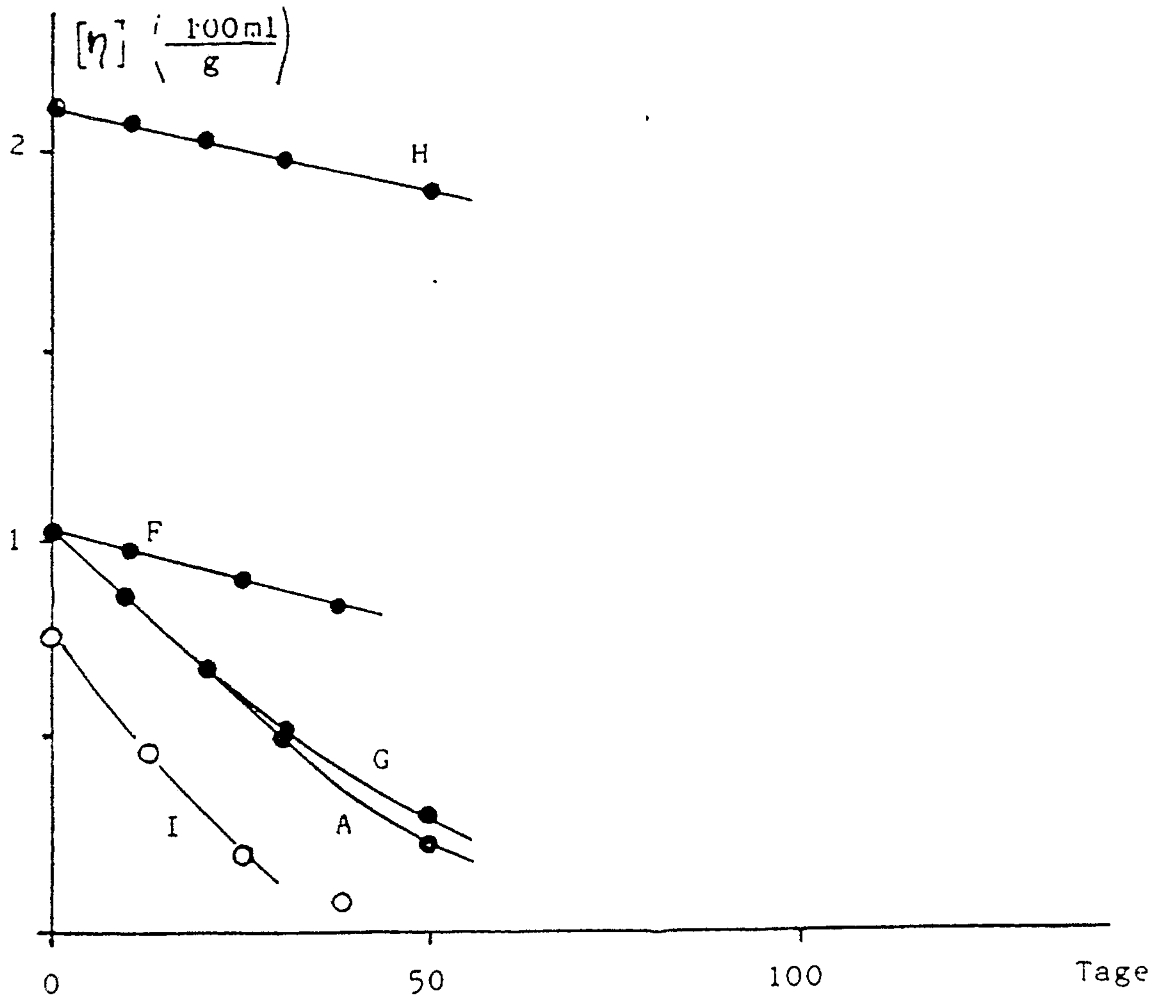


Abb. 3 Molmassenabnahme von Polylaktid-Formkörpern

Versuchsbedingungen in vitro: isotonischer Phosphatpuffer: pH 7,4; 37° C.
Zubereitung: Filmrollen Lösungsmethode)
A: D,L-Polylaktid I; 7 % Essigester; Tg = 26° C
F: D,L-Polylaktid I; 1 % Essigester; Tg = 48° C
G: D,L-Polylaktid I; 4 % Essigester; Tg = 35° C
H: D,L-Polylaktid II; 1 % Essigester; Tg = 52° C
I: D,L-Polylaktid II + 50 % Polymilchsäure (D,L); 1 % Essigester; Tg = 30° C

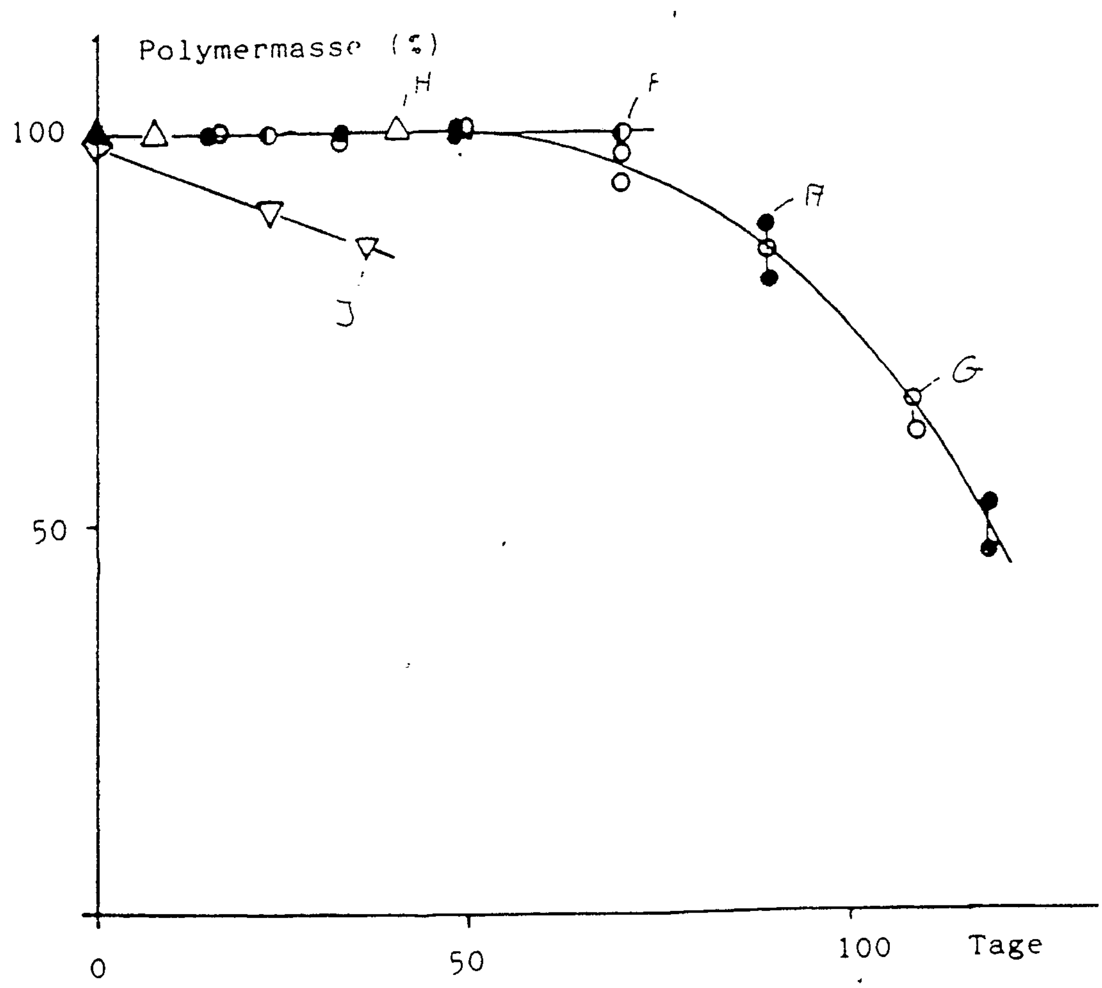


Abb. 4: Massenabnahme von dl = Polylaktid-Formkörpern

in vitro Versuchsbedingungen: isot. Phosphatpuffer; pH 7,4 %; 37 °C
Zubereitung: Filmrollen (Lösungsmethode)
A: D,L-Polylaktid I; 7 % Essigester; Tg = 26 °C
F: D,L-Polylaktid I; 1 % Essigester; Tg = 48 °C
G: D,L-Polylaktid I; 4 % Essigester; Tg = 35 °C
H: D,L-Polylaktid II; 1 % Essigester; Tg = 52 °C
I: D,L-Polylaktid II + 50 % Polymilchsäure 1 % Essigester; Tg = 30 °C

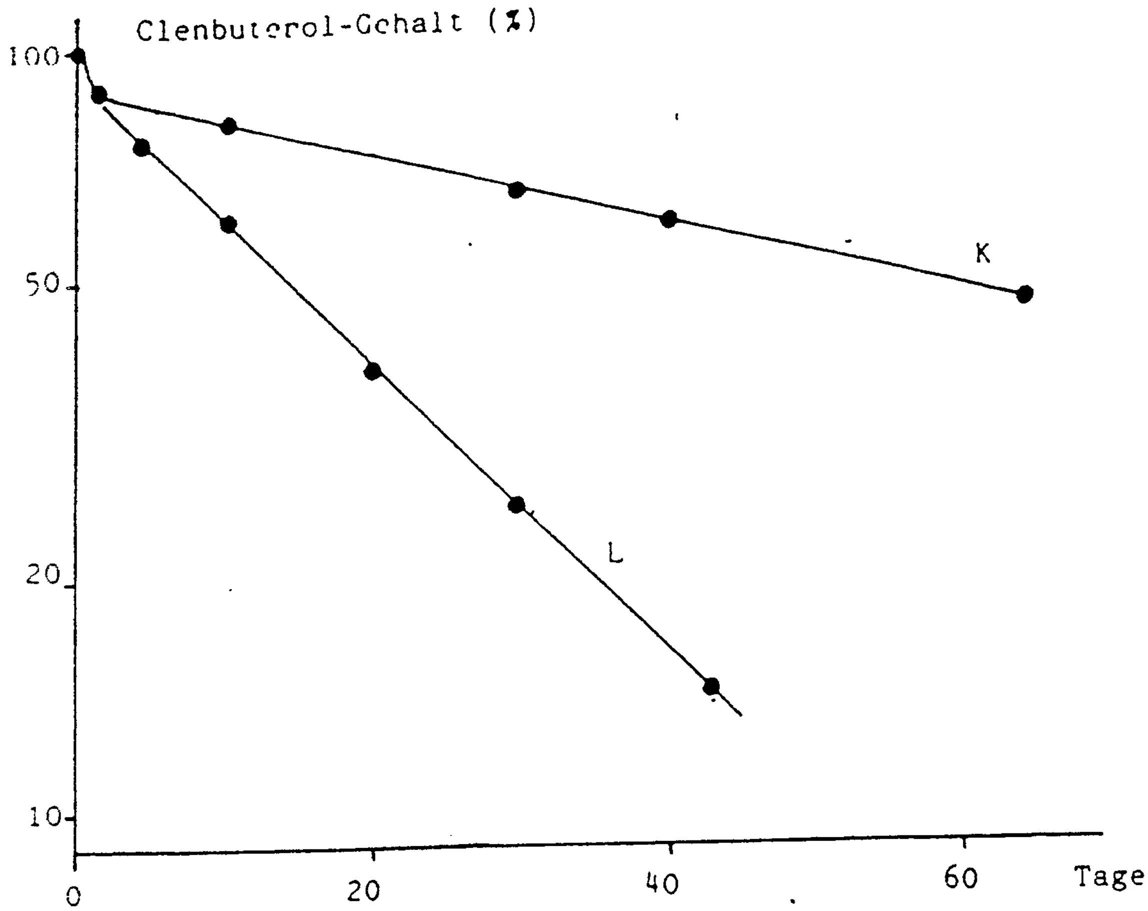


Abb. 5: Clenbuterol-Freigabe aus D,L-Polylaktid-Formkörpern

Versuchsbedingungen: isoton. Phosphatpuffer; pH 7,4; 37° C
Zubereitung: 3-Schichten-Filmrollen ([η] = 2,2 (100 ml/g)) mit 23,5 Gew. % Glenbuterol HCl und 4 % Essigester

| | Laktose (Gew. %) | | |
|---|---|---|---|
| | 1. Schicht | 2.Schicht | 3. Schicht |
| K | 0 | 0 | 0 |
| L | 0 | 25 % | 0 |

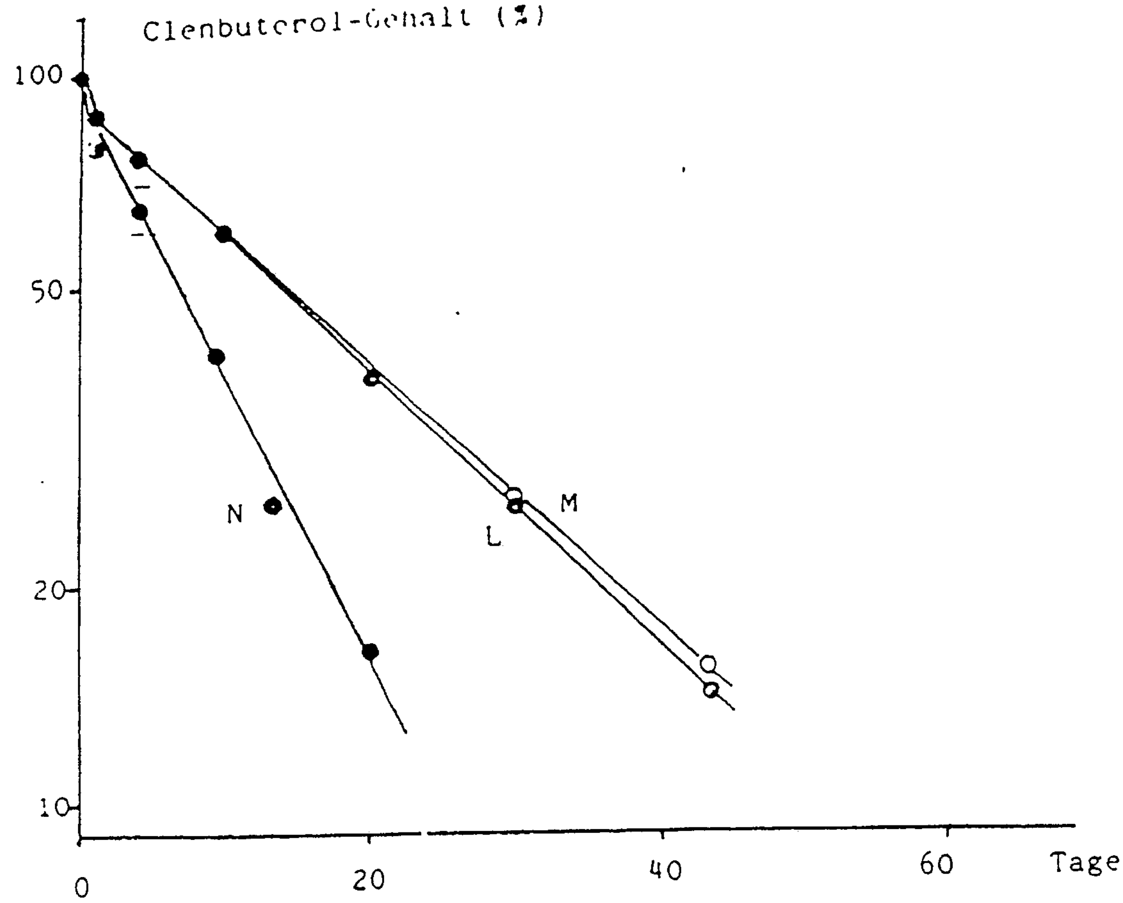


Abb. 6 Clenbuterol-Freigabe aus D,L-Polylaktid Formkörpern

in vitro Versuchsbedingungen isoton. Phosphatpuffer; pH 7,4; 37° C.

Zubereitung:

3-Schicht-Filmrollen mit 10 Gew.% Laktose und 23,5 Gew. % Clenbuterol HCl dl-Polylaktid II ([η] = 2,2 (100 ml/g))

Zusätze:

L: 4 % Essigester

M: 1 % Essigester

N: 1 % Essigester + 25 % dl-Polymilchsäure

**Ansprüche**

1) Oral verabreichbares Wirkstoffabgabesystem mit verzögerter Wirkstofffreigabe enthaltend einen wirkstoffhaltigen Teil und einen ein Beschwerungsmittel enthaltenen Teil, dadurch gekennzeichnet, daß das Beschwerungsmittel in Form kleiner Teilchen vorliegt, die durch einen in vorherbestimmbarer Zeit in physiologisch unbedenkliche Bestandteile zerfallenden Hilfsstoff verbunden sind.

2) Wirkstoffabgabesystem nach Anspruch 1, dadurch gekennzeichnet, daß der in vorherbestimmbarer Zeit zerfallende Hilfsstoff ein unter physiologischen Bedingungen abbaubares Polymer ist.

3) Wirkstoffabgabesystem nach Anspruch 1 oder 2, dadurch gekennzeichnet, daß das Beschwerungsmittel ein pulverförmiges Metall oder eine Metallverbindung mit einer Dichte größer als 3,0, bevorzugt größer als 4,0 g $cm^{-3}$, enthält.

4) Wirkstoffabgabesystem nach Anspruch 1, 2 oder 3, dadurch gekennzeichnet, daß das Polymer mit dem die Teilchen des Beschwerungsmittels verbunden sind ein Polymerisat oder Copolymerisat auf der Basis von Glycolid, Lactid, Dioxanon und/oder Trimethylencarbonat ist.

5) Wirkstoffabgabesystem nach einem der Ansprüche 1 bis 4, dadurch gekennzeichnet, daß das wirkstoffhaltige Teil den Wirkstoff retardiert freisetzt.

6) Wirkstoffabgabesystem nach Anspruch 5, dadurch gekennzeichnet, daß der Wirkstoff in einem unter physiologischen Bedingungen abbaubaren Polymer eingeschlossen ist.

7) Wirkstofabgabesystem nach Anspruch 5 und 6, dadurch gekennzeichnet, daß das Polymer ein Polymerisat oder Copolymerisat auf der Basis von Glycolid, L-Lactid, D-Lactid, D,L-Lactid, meso-Lactid, Dioxanon und/oder Trimethylencarbonat ist.

8) Wirkstoffabgabesystem nach Anspruch 5, dadurch gekennzeichnet, daß das Wirkstoff-Depot ein nicht abbaubares Trägermaterial enthält, welches in Form kleiner, ausscheidbarer Teilchen vorliegt.

9) Wirkstoffabgabesystem nach Anspruch 5 oder 6, dadurch gekennzeichnet, daß das wirkstoffenthaltende Teil ein Trägermaterial auf der Basis von Poly-D,L-Lactid mit darin eingearbeitetem Wirkstoff enthält, wobei das Trägermaterial definierte Anteile an Zusätzen in Form von Porenbildnern, bis ca. 10 Gew.-% eines physiologisch unbedenklichen Lösungsmittels oder Weichmachers und/oder niedermolekularer Polymilchsäure enthält.

10) Wirkstoffabgabesystem nach Anspruch 9, dadurch gekennzeichnet, daß das Trägermaterial ein Copolymerisat aus D,L-Lactid und Glykolid enthält, wobei der Glykolidanteil 50 Gew.% nicht überschreitet.

11) Wirkstoffabgabesystem nach Anspruch 9 oder 10, dadurch gekennzeichnet, daß das Trägermaterial bis zu 10 % Gew. eines Essigsäureesters enthält.

12) Wirkstoffabgabesystem nach einem der Ansprüche 5, 6, 9 bis 11, dadurch gekennzeichnet, daß das Trägermaterial niedermolekulare Polymilchsäure und/oder Lactose enthält.

13) Wirkstoffabgabesystem nach einem der vorhergehenden Ansprüche 1 bis 12, dadurch gekennzeichnet, daß es aus einem das Beschwerungsmittel enthaltenen mit kleinen Öffnungen versehenen Hohlkörper und einem darin eingeschlossenen ein- oder mehrteiligen Wirkstoff-Depot besteht, wobei das Wirkstoff-Depot eine Kombination mehrerer Arzneimittel enthalten kann.

14) Verfahren zur Herstellung eines Wirkstoffabgabesystems nach einem der Ansprüche 1 bis 13, dadurch gekennzeichnet, daß man dieses aus einem Beschwerungsmittel bestehend aus kleinen Teilchen mit einer Dichte größer als 3,0 g $cm^{-3}$, einem diese Teilchen verbindenden Hilfsstoff und einem Wirkstoff-Depot herstellt.

15) Verwendung eines Wirkstoffabgabesystems gemäß einem der Ansprüche 1 bis 13 bei Wiederkäuern zur Prophylaxe und/oder zur Behandlung von Krankheiten.

16) Verwendung eines Wirkstoffabgabesystems gemäß einem der Ansprüche 1 bis 13 bei Wiederkäuern zur Beeinflussung des Wachstums, des Stoffwechsels, des Körpergewichts, der Gewebezusammensetzung und/oder Futterverwertung.

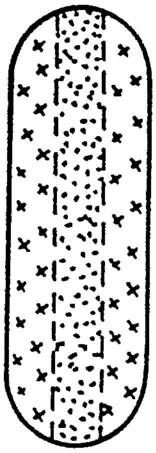

FIG.1.

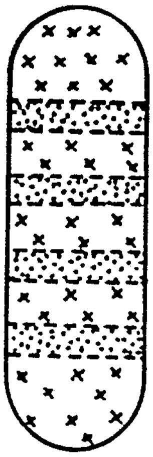

FIG.2.

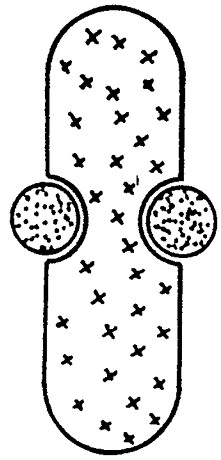

FIG.3A.

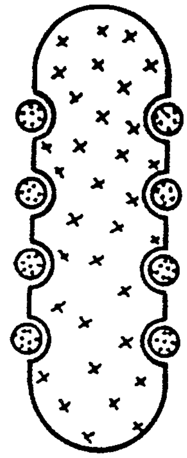

FIG.3B.

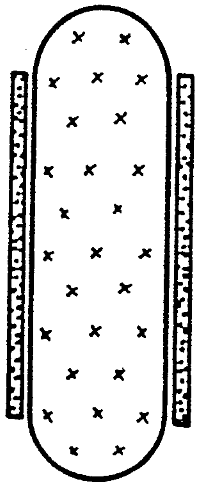

FIG.4.

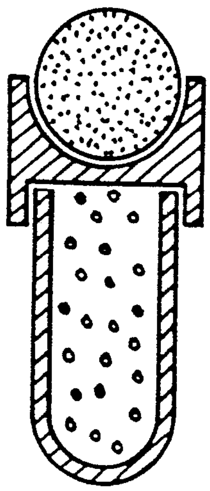

FIG.5.

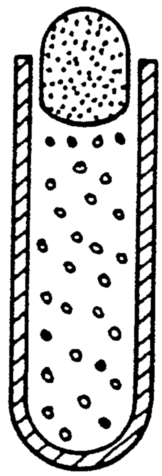

FIG.6.

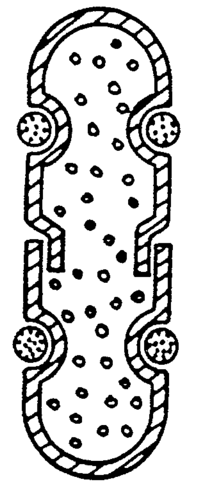

FIG.7A.

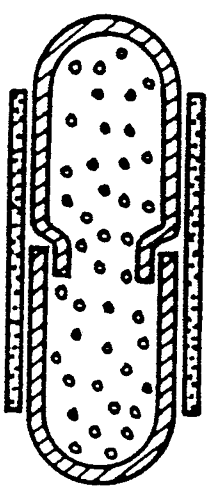

FIG.7B.

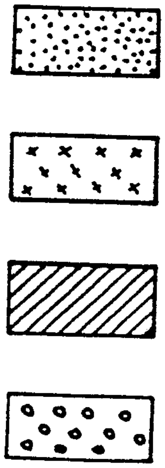

Mischung aus abbaubarem Polymer und Wirkstoff.

Mischung aus abbaubarem Polymer und Teilchen hoher Dichte.

Abbaubares Polymer

Teilchen hoher Dichte (Beschwerungsmittel)

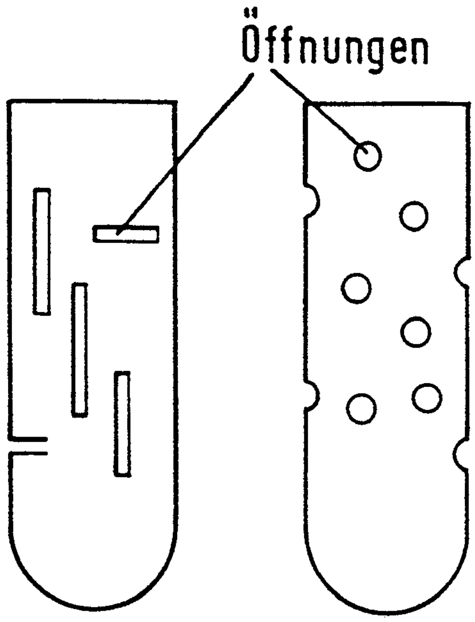


FIG.8A. FIG.8B.

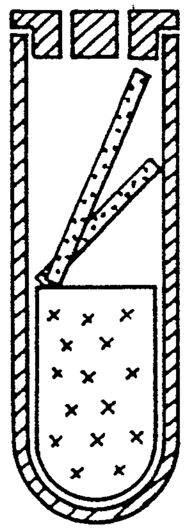

FIG.9.

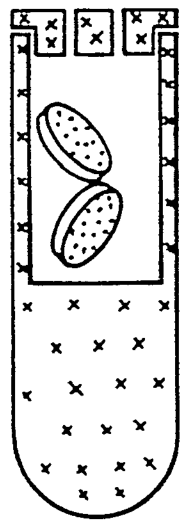

FIG.10.

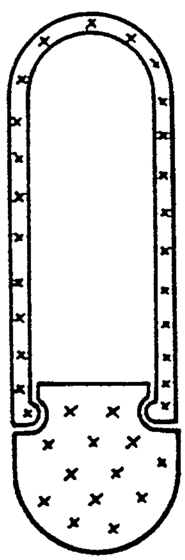

FIG.11.

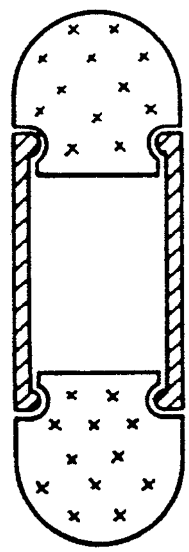

FIG.12.

Europäisches Patentamt

# EUROPÄISCHER RECHERCHENBERICHT

Nummer der Anmeldung

EP 89 10 3832

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-2 077 103 (PITMANN-MOORE) * Ansprüche 1,2,7; Seite 2, linke Spalte, Zeilen 10-25; Seite 3, rechte Spalte, Zeilen 113-122 * | 1-7,15, 16 | A 61 K 9/22 |
| D,A | EP-A-0 164 241 (ELI LILLY) * Ansprüche 1-4,8-10 * | 1-7,15, 16 | |

| RECHERCHIERTE SACHGEBIETE (Int. Cl.4) |
|---|
| A 61 K |

Der vorliegende Recherchenbericht wurde für alle Patentansprüche erstellt

| Recherchenort | Abschlußdatum der Recherche | Prüfer |
|---|---|---|
| DEN HAAG | 09-06-1989 | SCARPONI U. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)